# EUROPEAN PATENT APPLICATION

(11) **EP 4 488 251 A1**
(43) Date of publication of application: **08.01.2025**
(21) Application number: 23852689.1
(22) Date of filing: 25.05.2023
(51) Int. Cl.: C07C 29/03, C07C 29/88, C07C 31/10

(54) **METHOD AND APPARATUS FOR PREPARING ISOPROPYL ALCOHOL**

(30) Priority: 11.08.2022 KR 20220100549; 20.12.2022 KR 20220179081
(71) Applicant: LG Chem, Ltd., Yeongdeungpo-gu Seoul 07336 (KR)
(72) Inventor: JANG, Kyung Soo, Daejeon 34122 (KR); LEE, Sung Kyu, Daejeon 34122 (KR); HWANG, Sung June, Daejeon 34122 (KR); LIM, Kil Taek, Daejeon 34122 (KR)
(74) Representative: Goddar, Heinz J.
(86) International application number: PCT/KR2023/007150
(87) International publication number: WO 2024/034794

(57) **Abstract**

Provided are a method for preparing isopropyl alcohol including: (S1) supplying a feed stream including a propylene monomer and water to a reactor in the presence of a catalyst to perform a gas phase reaction; (S2) partially condensing a reaction product obtained by the gas phase reaction in a heat exchanger, transferring an uncondensed gas phase reaction product to an absorption column, and transferring a portion of the condensed liquid phase reaction product to a pH measurement system connected on-line between the heat exchanger and the absorption column; (S3) measuring a pH of the liquid phase reaction product transferred to the pH measurement system to calculate a catalyst content, and supplementing an amount of a catalyst corresponding to the calculated catalyst content to the reactor; and (S4) dissolving isopropyl alcohol included in the gas phase reaction product in absorbing water and separating the isopropyl alcohol in the absorption column, and an apparatus for performing the method.

## Description

### [Technical Field]

### CROSS-REFERENCE TO RELATED APPLICATIONS

The present application claims the benefit of priorities to Korean Patent Application No. 10-2022-0100549, filed on August 11, 2022, and Korean Patent Application No. 10-2022-0179081, filed on December 20, 2022, the entire contents of which are incorporated herein as a part of the specification.

### Technical Field

The present invention relates to a method and an apparatus for preparing isopropyl alcohol, and more particularly, to a method and an apparatus for preparing isopropyl alcohol which may maintain reactor performance by estimating a catalyst loss and supplementing the catalyst in real time in a reactor.

### [Background Art]

Isopropyl alcohol (IPA) is used for various purposes including a solvent for cleaning agents, raw materials for industrial paints and reagents, paints, inks, and the like in the electronics industry such as manufacture of semiconductor or liquid crystal displays (LCD).

Isopropyl alcohol may be prepared by reacting propylene and water. For example, a propylene monomer and water are reacted in a gas phase under high pressure and high temperature conditions in the presence of an acid catalyst in a reactor, thereby obtaining a reaction product including isopropyl alcohol, an unreacted propylene monomer, and water; the gas phase reaction product moves to an absorption column through a heat exchanger; a gas phase stream including an unreacted propylene monomer is discharged in the upper portion and a mixture of isopropyl alcohol and water is separated in the lower portion of the absorption column; and then isopropyl alcohol may be obtained by purification from the mixture. Water separated from the mixture may be recycled as process water and used, and a portion thereof is discharged as wastewater.

As the acid catalyst, a phosphoric acid-based catalyst such as SiO₂-supported H₃PO₄ may be used, and as a portion of the phosphoric acid is introduced to a reaction product, phosphoric acid loss may occur in the reactor, and thus, reaction efficiency is deteriorated to decrease a final yield of isopropyl alcohol.

Thus, a process of estimating a phosphoric acid loss in the reactor and supplementing phosphoric acid for maintaining reaction efficiency is needed.

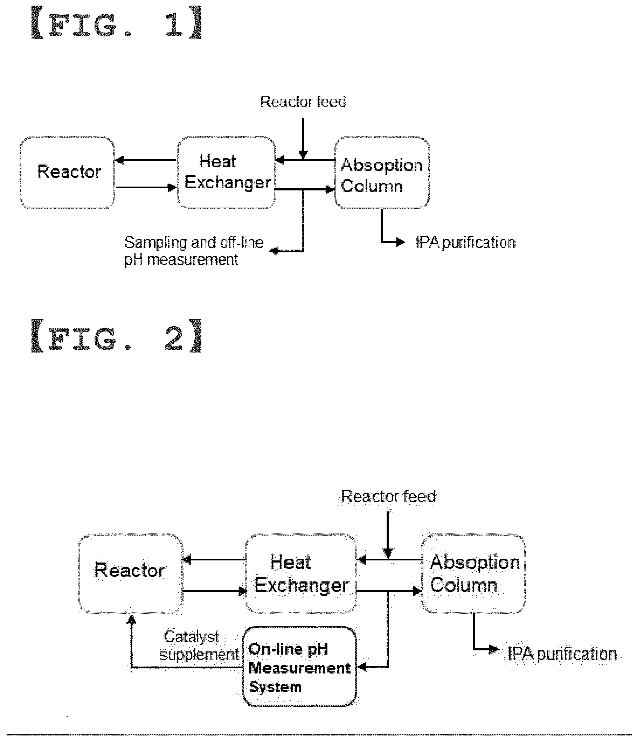

FIG. 1 schematically shows a process of estimating a catalyst loss performed in a conventional process of preparing isopropyl alcohol, in which a portion of a stream is taken in a pipe between a heat exchanger and an absorption column and sampled to obtain a sample solution, of which the pH is measured, and a phosphoric acid loss in the reactor may be estimated from the result and supplemented.

In the conventional technology as described above, since a reaction product at a high pressure is sampled and the pH of the sample is measured off-line, environmental and safety difficulties such as steam generation follow in the sampling process.

### [Disclosure]

### [Technical Problem]

The present invention is to solve the problems mentioned in the Background Art, and an object of the present invention is to provide a method and an apparatus which may estimate and supplement a catalyst loss in real time for maintaining reaction efficiency of propylene and water during preparation of isopropyl alcohol.

### [Technical Solution]

In one general aspect, a method for preparing isopropyl alcohol includes:
(51) supplying a feed stream including a propylene monomer and water to a reactor in the presence of a catalyst to perform a gas phase reaction;
(S2) partially condensing a reaction product obtained by the gas phase reaction in a heat exchanger, transferring an uncondensed gas phase reaction product to an absorption column, and transferring a portion of the condensed liquid phase reaction product to a pH measurement system connected on-line between the heat exchanger and the absorption column;
(S3) measuring a pH of the liquid phase reaction product transferred to the pH measurement system to calculate a catalyst content, and supplementing an amount of the catalyst corresponding to the calculated content to the reactor; and
(S4) dissolving isopropyl alcohol included in the gas phase reaction product in absorbing water and separating the isopropyl alcohol in the absorption column.

In another general aspect, an apparatus for preparing isopropyl alcohol includes:
a reactor in which a propylene monomer and water are reacted in the presence of a catalyst;
a heat exchanger which partially condenses a gas phase reaction product obtained from the reactor;
an absorption column which separates isopropyl alcohol included in the gas phase reaction product transferred from the heat exchanger; and
a pH measurement system which is connected on-line between the heat exchanger and the absorption column.

### [Advantageous Effects]

According to the present invention, in order to maintain reaction efficiency of propylene and water during preparation of isopropyl alcohol, the pH of the reaction product is measured on-line and the lost catalyst content is calculated from the result, thereby estimating and supplementing a catalyst loss in real time to maintain reaction efficiency to produce isopropyl alcohol.

In addition, since the on-line pH measurement as such does not involve a separate sampling process as in a conventional off-line method, steam generation is suppressed, which is advantageous from the environmental and safety point of view.

### [Description of Drawings]

FIG. 1 schematically shows a process of sampling a portion of a reaction product and then measuring a pH off-line during a conventional preparation process of isopropyl alcohol.
FIG. 2 schematically shows a process of preparing isopropyl alcohol including a step of measuring a pH of the reaction product according to an exemplary embodiment of the present invention on-line.
FIG. 3 shows an example in which an on-line pH measurement system is applied in a process of measuring isopropyl alcohol according to an exemplary embodiment of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The meaning of "comprising" or "containing" used herein embodies specific characteristics, domains, integers, steps, actions, elements, or components, and does not exclude the addition of other specific characteristics, domains, integers, steps, actions, elements, or components.

The term "stream" used herein may refer to a fluid flow in a process, or may refer to a fluid itself flowing in a pipe. Specifically, the stream may refer to both a fluid itself flowing in a pipe connecting each device and a fluid flow. In addition, the fluid may include any one or more components of gas, liquid, and solid.

An exemplary embodiment of the present invention relates to a method for preparing isopropyl alcohol (IPA), and specifically, includes (S1) supplying a feed in the presence of a catalyst and reacting it, (S2) partially condensing and transferring the reaction product, (S3) measuring a pH of a liquid phase reaction product obtained by partially condensation on-line, and (S4) separating isopropyl alcohol from the reaction product.

FIG. 2 schematically shows a process of preparing isopropyl alcohol including a step of measuring a pH of the reaction product according to an exemplary embodiment of the present invention on-line, and hereinafter, referring to FIG. 2, the method for preparing isopropyl alcohol according to the present invention will be described step by step.

First, a reactor is filled with a catalyst, and then a feed stream including a propylene monomer and water is supplied to the reactor and a gas phase reaction is performed, thereby obtaining a gas phase stream including isopropyl alcohol as a reaction product, an unreacted propylene monomer, unreacted water, the catalyst used in the reaction, and impurities (S1).

In the feed stream supplied to the reactor, a mole ratio of water to the propylene monomer may be 0.3 to 0.5, 0.35 to 0.5, or 0.35 to 0.45. When the mole ratio is satisfied, a forward reaction of an equilibrium reaction is promoted and a reward reaction is prevented from progressing, thereby increasing a yield of isopropyl alcohol.

The reactor may be operated under optimal conditions in which isopropyl alcohol may be efficiently prepared by the gas phase reaction of a propylene monomer and water. For example, an operation pressure of the reactor may be 30 to 50 kg/cm²·g, 35 to 50 kg/cm²·g, or 35 to 45 kg/cm²·g, and an operation temperature thereof may be 180 to 220°C, 185 to 220°C, or 185 to 215°C.

The catalyst is for promoting the reaction of propylene and water which are used as raw materials to improve a conversion rate from propylene to isopropyl alcohol and the final yield of isopropyl alcohol therefrom, and an acid catalyst, for example, a phosphorus acid-based catalyst may be used.

In order to increase the gas phase reaction efficiency of propylene and water, a catalyst life should be increased, and to this end, a portion of phosphoric acid which is an effective component of the catalyst is introduced to the reaction product to cause a phosphoric acid loss in the reactor. In order to supplement the catalyst lost in the reactor, a phosphoric acid component should be continuously or discontinuously supplemented, and the amount of further supplemented phosphoric acid may be determined by analyzing the pH of an effluent after the reaction.

To this end, in the method for preparing isopropyl alcohol according to the present invention, a gas phase reaction product obtained in the reactor is transferred to a heat exchanger and partially condensed, an uncondensed gas phase reaction product is transferred to an absorption column, and a portion of the condensed liquid phase reaction product is transferred to a pH measurement system connected on-line between the heat exchanger and the absorption column (S2).

Specifically, the reaction product obtained under high pressure and high temperature conditions of the reactor is partially condensed by heat exchange with the feed stream supplied to the reactor and may be in the form of a liquid phase or gas phase mixture. That is, gas-liquid separation of the reaction product is performed in the heat exchanger.

For example, the temperature of the feed stream before passing through the heat exchanger may be 90 to 130°C, 95 to 130°C, or 95 to 125°C, and a temperature of the feed stream after passing through the heat exchanger may be 160 to 210°C, 165 to 210°C, or 170 to 200°C. In addition, the temperature of the stream condensed by passing through the heat exchanger may be 100 to 140°C, 110 to 140°C, or 110 to 130°C.

If necessary, the stream condensed by passing through the heat exchanger may be further condensed using a cooler.

As such, the reaction product is heat exchanged with the feed stream, thereby being converted into a stream of a gas-liquid mixture, and also, the feed stream is preheated and supplied to the reactor, thereby reducing energy for heating the feed stream.

The gas-liquid mixed phase obtained by partial condensation by the heat exchange may be separated into a gas phase and a liquid phase in the heat exchanger itself, or may be separated into a gas phase and a liquid phase after further installing a separator in the latter stage of the heat exchanger.

Subsequently, the separated gas phase reaction product stream is transferred to an absorption column, and a portion of the liquid phase reaction product is transferred to a pH measurement system connected on-line between the heat exchanger and the absorption column to measure the pH, in order to figure out the phosphoric acid content of the introduced catalyst (S3).

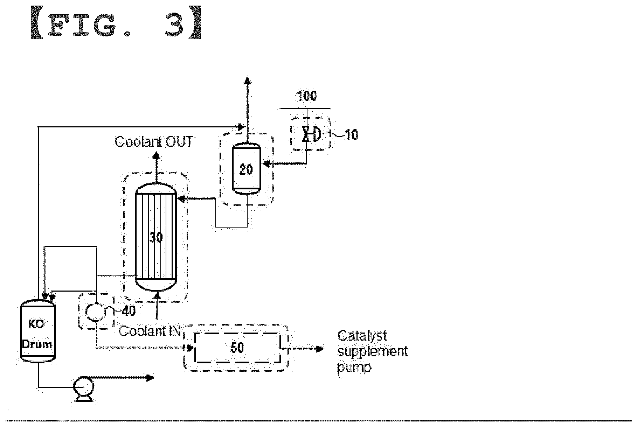

FIG. 3 shows an example in which an on-line pH measurement system is applied in a process of measuring isopropyl alcohol according to an exemplary embodiment of the present invention, and the pH measurement system may include a depressurization unit 10, a gas removal unit 20, a cooling unit 30, a pH measurement unit 40, and an arithmetic unit 50. When the on-line pH measurement system as such is used, a separate sampling process which is conventionally performed in pH measurement of the reaction product may be omitted.

Referring to FIG. 3, a portion of a stream 100 of the liquid phase reaction product obtained by partial condensation by heat exchanger is transferred to a depressurization unit 10 of the pH measurement system connected by a line between the heat exchanger and the absorption column. Herein, the liquid phase reaction product may be transferred through pressure control using a valve means installed on a line.

In the depressurization unit 10 of the pH measurement system, since the liquid phase reaction product is obtained by condensation in the heat exchanger in the latter stage of the reactor and a high-pressure stream of about 30 kg/cm²·g which is the pressure of the latter stage of the reactor is shown, depressurization for adjusting the pressure of the liquid reaction product to a pressure range required in the pH measurement system is performed.

For example, a portion of the liquid phase stream obtained by partial condensation in the heat exchanger is transferred at a flow rate of 15 to 100 L/h, and the pressure of the transferred stream in the depressurization unit included in the pH measurement system may be lowered to a range of 1/25 to 1/35 of the initial pressure, for example, 1 kg/cm²·g which is a normal pressure level.

Meanwhile, gas may be produced in the depressurization process of the liquid phase stream, and when the gas is introduced to the liquid phase stream, an error may occur in pH measurement, and thus, gas removal is needed.

Therefore, the depressurized liquid phase stream is passed through the gas removal unit 20 of the pH measurement system to remove gas, thereby preventing a gas component from being introduced to the liquid stream in the subsequent cooling process. The gas removal unit 20 may be in the form of a separator which separates a flowing fluid having two or more phases like a knock out drum, but is not limited thereto.

The degassed liquid phase stream is transferred to a cooling unit 30 of the pH measurement system, and is supplied with a refrigerant (for example, coolant) in the lower portion of the cooling unit 30, transferred to the upper portion, and discharged, thereby lowering the temperature of the liquid phase stream to a range of 20 to 40°C.

The pH of the cooled liquid phase stream is measured in the pH measurement unit 40, the loss of the phosphoric acid-based catalyst introduced to the reaction mixture is calculated from the result of pH measurement in a arithmetic unit, and the amount of the phosphoric acid-based catalyst corresponding to the calculated catalyst content is supplemented in the reactor, thereby increasing the life of the catalyst in the reactor to secure the final yield of isopropyl alcohol. Herein, catalyst supplement may be performed by controlling a pump flow rate of the catalyst supply line connected to the reactor.

In addition, the liquid phase stream which is passed through the cooling unit 3 and the pH measurement unit 40 may be transferred to a separate separator, for example, a knock out drum (KO drum), and a small amount of gas components remaining in the separate separator may be discharged with gas components removed in the gas removal unit 20.

Meanwhile, after gas-liquid separation in the heat exchanger, the gas phase reaction product is transferred to the absorption column, isopropyl alcohol included in the reaction product is dissolved using absorbing water in the absorption column and separated to the lower portion, and a stream including an unreacted propylene monomer included in the reaction product may be separated to the upper portion (S4) .

In an exemplary embodiment, the absorption column may be operated under conditions in which the upper stream including the unreacted propylene monomer and the lower stream including isopropyl alcohol may be effectively separated, and for example, the operation pressure of the absorption column may be in a range of 20 to 40 kg/cm²·g, 25 to 40 kg/cm²·g, or 25 to 35 kg/cm²·g and the operation temperature may be 80 to 110°C, 90 to 110°C, or 90 to 100°C.

The stream including isopropyl alcohol and water separated in the lower portion of the absorption column is supplied to a purification unit to separate water, thereby obtaining high-purity isopropyl alcohol. Water separated in the purification unit may be recovered as process water and reused, or discharged as wastewater.

Meanwhile, the stream including an unreacted propylene monomer separated in the upper portion of the absorption column may include impurities such as inert gas, and in order to use the unreacted propylene monomer in the preparation of isopropyl alcohol again, the stream may be passed through an inert gas removal column.

If necessary, in the method for preparing isopropyl alcohol according to the present invention, a device such as a distillation column, a condenser, a reboiler, a valve, a pump, a separator, and a mixer may be further installed and used.

According to the present invention, in order to maintain reaction efficiency of propylene and water during preparation of isopropyl alcohol, the pH of the reaction product is measured on-line and the catalyst content is calculated from the result, thereby estimating and supplementing a catalyst loss in real time to maintain the yield of isopropyl alcohol.

In addition, since the on-line pH measurement as such does not involve a separate sampling process as in a conventional off-line method, steam generation is suppressed, which is advantageous from the environmental and safety point of view.

The method for preparing isopropyl alcohol of the present invention as described above may be performed using an apparatus including a pH measurement system connected on-line.

Therefore, the present invention further provides an apparatus for preparing isopropyl alcohol including: a reactor in which a propylene monomer and water are reacted in the presence of a catalyst; a heat exchanger which partially condenses a gas phase reaction product obtained from the reactor; an absorption column which separates isopropyl alcohol included in the gas phase reaction product transferred from the heat exchanger; and a pH measurement system which is connected on-line between the heat exchanger and the absorption column.

In the preparation apparatus, descriptions for the constituents overlapping those in the preparation method are the same.

## Claims

1. A method for preparing isopropyl alcohol, including:
(S1) supplying a feed stream including a propylene monomer and water to a reactor in the presence of a catalyst to perform a gas phase reaction;
(S2) partially condensing a reaction product obtained by the gas phase reaction in a heat exchanger, transferring an uncondensed gas phase reaction product to an absorption column, and transferring a portion of the condensed liquid phase reaction product to a pH measurement system connected on-line between the heat exchanger and the absorption column;
(S3) measuring a pH of the liquid phase reaction product transferred to the pH measurement system to calculate a catalyst content, and supplementing an amount of the catalyst corresponding to the calculated catalyst content to the reactor; and
(S4) dissolving isopropyl alcohol included in the gas phase reaction product in absorbing water and separating the isopropyl alcohol in the absorption column.

2. The method for preparing isopropyl alcohol of claim 1, wherein the gas phase reaction product is a gas phase stream including isopropyl alcohol, an unreacted propylene monomer, unreacted water, a catalyst used in the reaction, and impurities.

3. The method for preparing isopropyl alcohol of claim 1, wherein the catalyst includes a phosphoric acid-based catalyst.

4. The method for preparing isopropyl alcohol of claim 1, wherein the gas phase reaction product is condensed to 100 to 140°C by heat exchange in the heat exchanger with the feed stream being supplied to the reactor.

5. The method for preparing isopropyl alcohol of claim 1, wherein the portion of the condenced liquid phase reaction product obtained by condensation in the heat exchanger is transferred to the pH measurement system connected on-line by pressure control.

6. The method for preparing isopropyl alcohol of claim 1, wherein the pH of the liquid phase reaction product transferred to the pH measurement system is measured after depressurization, gas removal, and cooling processes thereof.

7. The method for preparing isopropyl alcohol of claim 6, wherein the depressurization of the liquid phase reaction product is performed to a pressure of 1/25 to 1/35 of initial pressure when transferred to the pH measurement system.

8. The method for preparing isopropyl alcohol of claim 6, wherein the gas removal of the liquid phase reaction product is to remove gas produced in the depressurization.

9. The method for preparing isopropyl alcohol of claim 6, wherein the cooling of the liquid reaction product is performed to a temperature of 20 to 40°C using a coolant.

10. An apparatus for preparing isopropyl alcohol comprising:
a reactor in which a propylene monomer and water are reacted in the presence of a catalyst;
a heat exchanger which partially condenses a gas phase reaction product obtained from the reactor;
an absorption column which separates isopropyl alcohol included in the gas phase reaction product transferred from the heat exchanger; and
a pH measurement system which is connected on-line between the heat exchanger and the absorption column.

11. The apparatus for preparing isopropyl alcohol of claim 10, wherein the pH measurement system includes a depressurization unit, a gas removal unit, a cooling unit, a pH measurement unit, and an arithmetic unit.
